# EUROPÄISCHE PATENTSCHRIFT

(11) **EP 3 583 324 B1**
(45) Veröffentlichungstag und Bekanntmachung des Hinweises auf die Patenterteilung: **01.09.2021**
(21) Anmeldenummer: 18707303.6
(22) Anmeldetag: 15.02.2018
(51) Int. Cl.: F16C 32/04

(54) **RÖNTGEN-COMPUTERTOMOGRAPHIEVORRICHTUNG UND VERFAHREN ZUM BETRIEB EINER RÖNTGEN-COMPUTERTOMOGRAPHIEVORRICHTUNG**
X-RAY COMPUTER TOMOGRAPHIC DEVICE AND METHOD FOR OPERATING A X-RAY COMPUTER TOMOGRAPHIC DEVICE
DISPOSITIF DE SCANNER TOMOGRAPHIQUE ET PROCÉDÉ D'UTILISATION D'UN TEL DISPOSITIF

(30) Priorität: 17.02.2017 DE 102017103332
(43) Veröffentlichungstag der Anmeldung: 25.12.2019
(73) Patentinhaber: thyssenkrupp rothe erde Germany GmbH, 44137 Dortmund (DE); thyssenkrupp AG, 45143 Essen (DE)
(72) Erfinder: PANTKE, Klaus, 59759 Arnsberg (DE); STENZEL, Christopher, 59494 Soest (DE)
(74) Vertreter: thyssenkrupp Intellectual Property GmbH
(86) Internationale Anmeldenummer: PCT/EP2018/053818
(87) Internationale Veröffentlichungsnummer: WO 2018/149932

(56) Entgegenhaltungen:
- DE-A1-102014 214 511
- DE-A1-102015 108 081
- DE-T2- 68 924 691
- JP-A- H1 137 155
- US-A- 5 109 659

## Beschreibung

### Stand der Technik

Die vorliegende Erfindung betrifft eine Röntgen-Computertomographievorrichtung, bei der eine Röntgenquelle und/oder ein Röntgendetektor gegenüber einem zu untersuchenden Objekt über ein Magnetlager mit einem ersten Lagerring und einem konzentrisch zu dem ersten Lagerring angeordneten zweiten Lagerring bewegbar gelagert ist, wobei der erste Lagerring und der zweite Lagerring mittels Elektromagneten um eine Drehachse drehbar zueinander gelagert sind, wobei der erste Lagerring eine erste Magnetreihe und eine zweite Magnetreihe aufweist, wobei die Magnetreihen jeweils in einer Umfangsrichtung des ersten Lagerrings zueinander beabstandet angeordnete Elektromagnete aufweisen. Ferner betrifft die Erfindung ein Verfahren zum Betrieb einer solchen Röntgen-Computertomographievorrichtung.

Derartige Magnetlager werden zur kontaktlosen Lagerung zueinander drehbarer Maschinenelemente verwendet und bieten gegenüber Wälz- oder Gleitlagern den Vorteil, dass besonders hohe Drehgeschwindigkeiten ermöglicht werden können. Zudem können Magnetlager nahezu lautlos betrieben werden.

Ein Magnetlager der oben genannten Art ist beispielsweise aus der DE 10 2015 108 081 A1 bekannt. Dieses Magnetlager weist einen Innenring sowie einen Außenring auf, die um eine Drehachse drehbar zueinander gelagert sind. In dem Innenring sind insgesamt drei Magnetreihen vorgesehen, welche jeweils aus mehreren in Umfangsrichtung des Innenrings zueinander beabstandet angeordneten Elektromagneten gebildet sind. Die Elektromagnete einer ersten Magnetreihe sind derart angeordnet, dass sie eine magnetische Kraft auf den Außenring bewirken, die in radialer Richtung wirkt. Diese Elektromagnete bilden ein magnetisches Radial-Teillager. Die Elektromagnete der beiden anderen Magnetreihen sind derart angeordnet, dass sie magnetische Kräfte in axialer Richtung auf den Außenring ausüben. Somit bilden diese Elektromagneten magnetische Axial-Teillager.

Das bekannte Magnetlager hat sich in der Praxis durchaus bewährt. Allerdings hat es sich als nachteilig herausgestellt, dass es eine hohe Anzahl an Elektromagneten aufweist, wodurch sich hohe Fertigungskosten ergeben.

Aus JPH1137155A ist ein Magnetlager zur Lagerung eines Rotors einer großen Strömungsmaschine, insbesondere einer Flugzeugturbine, bekannt. US 5 109 659 A beschreibt einen magnetischen Ring und DE 689 24 691 T2 eine Aufwickelvorrichtung mit einem rotierenden Ring, die beim Spinnen eines Garns eingesetzt werden.

DE102014214511 zeigt eine Röntgen-Computertomographievorrichtung gemäß dem Oberbegriff des Anspruchs 1 und ein Verfahren zum Betrieb einer solchen Vorrichtung gemäß dem Oberbegriff des Anspruchs 15.

### Offenbarung der Erfindung

Vor diesem Hintergrund ist es die Aufgabe der Erfindung, die Fertigung eines Magnetlagers mit reduzierten Kosten zu ermöglichen.

Diese Aufgabe wird gelöst durch ein Magnetlager mit einem ersten Lagerring und einem konzentrisch zu dem ersten Lagerring angeordneten zweiten Lagerring, wobei der erste Lagerring und der zweite Lagerring mittels Elektromagneten um eine Drehachse drehbar zueinander gelagert sind, wobei der erste Lagerring eine erste Magnetreihe und eine zweite Magnetreihe aufweist, wobei die Magnetreihen jeweils in einer Umfangsrichtung des ersten Lagerrings zueinander beabstandet angeordnete Elektromagnete aufweisen, wobei die Elektromagnete der Magnetreihen derart ausgerichtet sind, dass sie jeweils eine magnetische Kraft auf den zweiten Lagerring bewirken können, die quer zu der Drehachse und quer zu einer senkrecht zu der Drehachse angeordneten Radialebene ausgerichtet ist. Die Elektromagnete der ersten und zweiten Magnetreihe des erfindungsgemäßen Magnetlagers sind derart angeordnet, dass sie eine magnetische Kraft auf den zweiten Lagerring, insbesondere auf Wirkflächen des zweiten Lagerrings, bewirken können, die Kraftkomponenten sowohl in axialer Richtung als auch in radialer Richtung aufweist. Durch diese Anordnung ist es möglich, das Magnetlager mit lediglich zwei Magnetreihen auszubilden. Eine dritte Magnetreihe ist nicht erforderlich, so dass bei dem Magnetlager die Kosten für Elektromagnete reduziert werden können. Zudem können in dem ersten Lagerring eine geringere Anzahl an Aufnahmen für die Elektromagnete vorgesehen werden, wodurch die Fertigungskosten zusätzlich verringert werden.

Gemäß einer bevorzugten Ausgestaltung weist das Magnetlager außer den Elektromagneten der ersten Magnetreihe und der zweiten Magnetreihe keine weiteren Elektromagnete auf, so dass sich eine besonders kostengünstige Ausgestaltung ergibt.

Konstruktiv vorteilhaft ist es, wenn das Magnetlager permanentmagnetlos ausgebildet ist. Die magnetische Kraftwirkung auf den zweiten Lagerring kann dann ausschließlich von den Elektromagneten ausgehen.

Eine bevorzugte Ausgestaltung sieht vor, dass die Elektromagnete der Magnetreihen jeweils eine Spule umfassen, die um einen Spulenkern gewickelt ist, welcher eine Längsachse aufweist, die in Richtung eines Luftspalts zwischen dem ersten und dem zweiten Lagerring verläuft, wobei die Längsachse des Spulenkerns quer zu der Drehachse und quer zu der Radialebene ausgerichtet ist. Eine derartige Ausgestaltung der Elektromagnete erlaubt eine kompakte Ausgestaltung. Der Spulenkern ist bevorzugt einstückig mit dem ersten Lagerring ausgebildet. Alternativ ist es möglich, dass der Spulenkern lösbar mit dem ersten Lagerring verbunden ist, beispielsweise über eine Schraubverbindung. Der Spulenkern kann als Polschuh ausgebildet sein, über welchen das magnetische Feld der Spule im Luftspalt zwischen dem ersten und zweiten Lagerring ausgerichtet wird. Bevorzugt weist der Polschuh eine gekrümmte Oberfläche auf, welche an eine Krümmung des ersten Lagerrings angepasst ist.

Eine vorteilhafte Ausgestaltung sieht vor, dass die Elektromagnete der Magnetreihen derart ausgerichtet sind, dass eine gedachte Verlängerung der Längsachse des Spulenkerns eines Elektromagneten der ersten Magnetreihe ausgehend von dem ersten Lagerring und in Richtung des zweiten Lagerrings und eine gedachte Verlängerung der Längsachse des Spulenkerns eines Elektromagneten der zweiten Magnetreihe ausgehend von dem ersten Lagerring und in Richtung des zweiten Lagerrings eine zwischen der ersten und zweiten Magnetreihe angeordnete Radialebene schneiden. Eine derartige Anordnung hat sich insbesondere für solche Anwendungsfälle als vorteilhaft erwiesen, in denen das Magnetlager einer bezüglich einer mittleren Radialebene symmetrischen Lasteinleitung ausgesetzt ist.

Gemäß einer alternativen vorteilhaften Ausgestaltung sind die Elektromagnete der Magnetreihen derart ausgerichtet, dass eine gedachte Verlängerung der Längsachse des Spulenkerns eines Elektromagneten der ersten Magnetreihe ausgehend von dem ersten Lagerring und von dem zweiten Lagerring weg weisend und eine gedachte Verlängerung der Längsachse des Spulenkerns eines Elektromagneten der zweiten Magnetreihe ausgehend von dem ersten Lagerring und von dem zweiten Lagerring weg weisend eine zwischen der ersten und zweiten Magnetreihe angeordnete Radialebene schneiden. Im Vergleich zu einer Anordnung der Elektromagnete, bei welcher die jeweiligen Verlängerungen ausgehend von dem ersten Lagerring und in Richtung des zweiten Lagerrings die Radialebene zwischen den beiden Magnetreihen schneiden, kann bei dieser Anordnung der Elektromagnete eine verbesserte Momentenaufnahme und somit eine stabilere Lagerung ermöglicht werden. Bevorzugt weist der zweite Lagerring Wirkflächen auf, auf welche die magnetischen Kräfte der an dem ersten Lagerring angeordneten Elektromagneten einwirken, wobei die Wirkflächen quer zur Drehachse und quer zu der Radialebene angeordnet sind. Eine besonders kompakte Ausgestaltung des Magnetlagers kann erhalten werden, wenn der zweite Lagerring einen im Wesentlichen dreieckigen Querschnitt oder einen im Wesentlichen V-förmigen Querschnitt entlang einer axialen Schnittebene aufweist. Die Wirkflächen des zweiten Lagerrings sind bevorzugt derart angeordnet, dass die Flächennormalen der Wirkflächen zueinander einen Winkel im Bereich von 5° bis 175°, bevorzugt im Bereich von 30° bis 150°, besonders bevorzugt im Bereich von 40° bis 140° oder im Bereich von 45° bis 135° oder im Bereich von 50° bis 120° oder im Bereich von 80° bis 100°, beispielsweise von 90°, einschließen.

Der Betrag eines Neigungswinkels der Elektromagnete der ersten und zweiten Magnetreihe gegenüber der Radialebene, insbesondere der Spulenkerne der Elektromagnete der ersten und zweiten Magnetreihe gegenüber der Radialebene, kann im Bereich von 5° bis 85°, bevorzugt im Bereich von 15° bis 75°, besonders bevorzugt im Bereich von 30° bis 60°, beispielsweise bei 45°, liegen.

Erfindungsgemäß sind die Elektromagnete der ersten Magnetreihe bezüglich einer Radialebene asymmetrisch zu den Elektromagneten der zweiten Magnetreihe angeordnet und/oder die Elektromagnete der ersten Magnetreihe und die Elektromagnete der zweiten Magnetreihe sind unterschiedlich ausgelegt.

Beispielsweise können bei asymmetrischer Anordnung die Elektromagnete der ersten Magnetreihe bezüglich der Radialebene einen Versatz zu den Elektromagneten der zweiten Magnetreihe aufweisen, so dass entlang der Umfangsrichtung des Magnetlagers ein Elektromagnet der ersten Reihe versetzt zwischen zwei Elektromagneten der zweiten Magnetreihe angeordnet ist und umgekehrt. Eine bezüglich einer Radialebene asymmetrische Anordnung kann alternativ oder zusätzlich dadurch erreicht werden, dass die Elektromagnete der ersten Magnetreihe und der zweiten Magnetreihe, insbesondere die Spulenkerne der Elektromagnete der ersten Magnetreihe und die Spulenkerne der zweiten Magnetreihe, betragsmäßig unterschiedliche Neigungen gegenüber der Radialebene aufweisen. Hierdurch kann das Magnetlager an eine asymmetrische Lastsituation angepasst werden. Beispielsweise können die Elektromagnete der ersten Magnetreihe einen größeren Winkel gegenüber einer Radialebene aufweisen, um axiale Lasten in erhöhtem Maße abzufangen, und die Elektromagnete der zweiten Magnetreihe können einen kleineren Winkel gegenüber der Radialebene aufweisen, um radiale Lasten in erhöhtem Maße abzufangen. Eine Winkeldifferenz zwischen dem Neigungswinkel der Elektromagnete der ersten Magnetreihe gegenüber der Radialebene und dem Neigungswinkel der Elektromagnete der zweiten Magnetreihe gegenüber der Radialebene liegt bevorzugt im Bereich von 1° bis 30°, besonders bevorzugt im Bereich von 1° bis 15°, besonders bevorzugt im Bereich von 1° bis 10°, beispielsweise im Bereich von 1° bis 5°.

Durch unterschiedlich ausgelegte Elektromagnete der ersten und zweiten Magnetreihe kann die Anpassung der Elektromagnete an asymmetrische Lastsituationen vereinfacht werden. Auf diese Weise kann ein Magnetlager erhalten werden, bei dem die magnetischen Stützkräfte der ersten und zweiten Magnetreihe unterschiedlich stark ausgebildet sind. Beispielsweise können die Elektromagnete der ersten und zweiten Magnetreihe unterschiedliche Wicklungsanzahlen, eine unterschiedliche Größe der Spule und/oder des Spulenkerns aufweisen. Beispielsweise können die Elektromagnete der ersten und zweiten Magnetreihe eine unterschiedliche Breite und/oder unterschiedliche Höhe und/oder unterschiedliche Länge aufweisen. Vorteilhaft ist es, wenn das Verhältnis der Breite und/oder Höhe und/oder Länge der Elektromagnete der ersten und zweiten Magnetreihe im Bereich von 0,1 bis 0,9 liegt, bevorzugt im Bereich von 0,2 bis 0,5, besonders bevorzugt im Bereich von 0,3 bis 0,4. Bevorzugt unterscheiden sich die Elektromagnete der ersten und zweiten Magnetreihe hinsichtlich der Dimensionierung ihrer Polschuhe. Alternativ, jedoch nicht erfindungsgemäß ist es möglich, die Elektromagnete der ersten Magnetreihe und die Elektromagnete der zweiten Magnetreihe identisch auszulegen.

Gemäß einer vorteilhaften Ausgestaltung der Erfindung weist der zweite Lagerring eine Flusstrennung aus einem nicht-magnetischen Material auf. Über die Flusstrennung können die durch die Elektromagnete der ersten und zweiten Magnetreihe erzeugten Magnetkreise voneinander entkoppelt werden. Es ist damit möglich, die magnetischen Kräfte, die durch die Elektromagneten der ersten Magnetreihe und die Elektromagneten der zweiten Magnetreihe erzeugt werden, im Wesentlichen unabhängig voneinander einstellen. Bevorzugt ist die Flusstrennung ringförmig ausgebildet, beispielsweise als ringförmiges Flusstrennungselement. Die Flusstrennung kann im Bereich zwischen der ersten Magnetreihe und der zweiten Magnetreihe angeordnet sein. Das nicht-magnetische Material der Flusstrennung kann Aluminium, Austenit-Stahl, Bronze oder eine Keramik sein.

Vorteilhaft ist es, wenn das Magnetlager mindestens ein als Gleitlager ausgebildetes Fanglager aufweist. Über das Fanglager kann der jeweilige drehende Lagerring bei einem Ausfall der Stromversorgung der Elektromagnete gefangen werden, ohne dass eine mechanische Zerstörung des Lagers befürchtet werden muss. Das Fanglager kann ein erstes Fanglagerteil aufweisen, welches an dem ersten Lagerring angeordnet ist, und ein zweites Fanglagerteil, das an dem zweiten Lagerring angeordnet ist. Bevorzugt ist das erste Fanglagerteil in den ersten Lagerring integriert und/oder das zweite Fanglagerteil ist in den zweiten Lagerring integriert.

In diesem Zusammenhang hat es sich als vorteilhaft erwiesen, wenn das Fanglager zwischen der ersten und der zweiten Magnetreihe angeordnet ist. Durch das zwischen der ersten und der zweiten Magnetreihe angeordnete erste Fanglager kann das Magnetlager gefangen werden ohne dass weitere Fanglager erforderlich sind. Insofern handelt es sich um ein in axialer Schnittrichtung des Magnetlagers innen, insbesondere mittig, angeordnetes Fanglager. Das zwischen der ersten und zweiten Magnetreihe angeordnete Fanglager kann ein, insbesondere an dem zweiten Lagerring angeordnetes, Fanglagerteil aufweisen, dass aus einem nicht-magnetischen Material, insbesondere Aluminium, Austenit-Stahl, Bronze oder Keramik ausgebildet ist. Hierdurch kann erreicht werden, dass das Fanglagerteil zugleich als Flusstrennung wirkt.

Alternativ oder zusätzlich kann das Magnetlager ein zweites und ein drittes Fanglager aufweisen, die derart angeordnet sind, dass die erste Magnetreihe und die zweite Magnetreihe in Richtung der Drehachse gesehen zwischen dem zweiten und dem dritten Fanglager angeordnet ist. Hierdurch kann ein Aufbau ermöglicht werden, bei welchem die Fanglager in axialer Schnittrichtung außen angeordnet sind, bzw. die erste und zweite Magnetreihe innen, insbesondere mittig, angeordnet ist.

Eine vorteilhafte Ausgestaltung sieht vor, dass der erste Lagerring ein Außenring ist und der zweite Lagerring ein Innenring ist. Der Außenring kann konzentrisch außerhalb des Innenrings angeordnet sein. Es ist möglich, dass der Außenring feststehend ausgebildet ist und der Innenring gegenüber dem Außenring drehbar ist. Alternativ kann der Innenring feststehend ausgebildet sein und der Außenring drehbar gegenüber dem Innenring vorgesehen sein.

Eine alternative, vorteilhafte Ausgestaltung sieht vor, dass der erste Lagerring ein Innenring und der zweite Lagerring ein Außenring ist. Der Innenring kann konzentrisch innerhalb des Außenrings angeordnet sein. Auch bei einer derartigen Ausgestaltung ist es möglich, entweder den Außenring feststehend auszubilden und den Innenring drehbar gegenüber dem Außenring oder den Innenring feststehend auszubilden und den Außenring drehbar gegenüber dem Innenring.

Bevorzugt ist der erste Lagerring als geteilter Lagerring mit einem ersten ringförmigen Lagerringteil und einem zweiten ringförmigen Lagerringteil ausgebildet. Hierdurch kann die Montage des Magnetlagers erleichtert werden. Die Elektromagnete können zunächst an dem ersten Lagerringteil und dem zweite Lagerringteil montiert werden, bevor die Verbindung des ersten Lagerringteils mit dem zweiten Lagerringteil erfolgt. Optional kann der erste Lagerring weitere, insbesondere ringförmige, Lagerringteile aufweisen.

Bevorzugt ist der zweite Lagerring als geteilter Lagerring mit einem dritten ringförmigen Lagerringteil und einem vierten ringförmigen Lagerringteil ausgebildet. Zusätzlich kann der zweite Lagerring ein ringförmiges Fanglagerteil aufweisen, welches zwischen dem dritten Lagerringteil und dem vierten Lagerringteil angeordnet ist.

Gemäß einer bevorzugten Ausgestaltung weist der zweite Lagerring mehrere Bleche auf, die gegeneinander elektrisch isoliert sind, so dass die Bildung von Wirbelströmen in dem zweiten Lagerring abgeschwächt werden kann. Besonders bevorzugt sind die Bleche in axialer Richtung gegeneinander elektrisch isoliert. Insofern können die Bleche als ringförmige gegeneinander elektrisch isolierte Bleche ausgebildet sein. Alternativ ist es möglich, dass die Bleche in Umfangsrichtung oder in radialer Richtung gegeneinander elektrisch isoliert sind. Alternativ oder zusätzlich kann der erste Lagerring mehrere Bleche aufweisen, die gegeneinander elektrisch isoliert sind, insbesondere in axialer Richtung, in Umfangsrichtung oder in radialer Richtung. Hierdurch kann die Bildung von Wirbelströmen in dem ersten Lagerring abgeschwächt werden.

Als vorteilhaft hat sich ferner eine Ausgestaltung erwiesen, bei welcher die Elektromagnete einer Magnetreihe in einem Magnetmodul angeordnet sind. Das Magnetmodul kann an dem ersten Lagerring angeordnet sein. Eine derartige Ausgestaltung bietet den Vorteil, dass das Magnetmodul vorgefertigt und dann an dem ersten Lagerring montiert werden kann. Bevorzugt ist das Magnetmodul ringförmig ausgebildet. Das Magnetmodul kann sämtliche Elektromagnete einer Magnetreihe aufnehmen. Alternativ können mehrere Magnetmodule mit jeweils mehreren Elektromagneten zusammen eine Magnetreihe bilden.

Das vorstehend beschriebene Magnetlager kann als elektrischer Antrieb Verwendung finden. Insofern kann durch das Magnetlager ein wälzlagerloser und gleitlagerloser Elektromotor bereitgestellt werden, bei welchem die Lagerung des jeweiligen drehenden Lagerrings berührungslos allein durch magnetische Kräfte erfolgt. Die jeweiligen Elektromagnete einer Magnetreihe können gleichzeitig Kräfte zur magnetischen Lagerung und zum Antrieb erzeugten. Alternativ ist es möglich, dass die Magenreihen Lager-Elektromagnete aufweisen, die Kräfte zur magnetischen Lagerung erzeugen und Antriebs-Elektromagnete, die Kräfte zum Antrieb erzeugen.

Ein weiterer Gegenstand der Erfindung ist eine Röntgen-Computertomographievorrichtung mit einem vorstehend beschriebenen Magnetlager. Über das Magnetlager kann eine Röntgenquelle und/oder ein Röntgendetektor gegenüber einem zu untersuchenden Objekt, beispielsweise einem Gegenstand oder einer Person, bewegbar gelagert werden.

Zur Lösung der eingangs genannten Aufgabe wird ferner ein Verfahren zum Betrieb eines Magnetlagers mit einem ersten Lagerring und einem konzentrisch zu dem ersten Lagerring angeordneten zweiten Lagerring vorgeschlagen, wobei der erste Lagerring und der zweite Lagerring mittels Elektromagneten um eine Drehachse drehbar zueinander gelagert sind, wobei der erste Lagerring eine erste Magnetreihe und eine zweite Magnetreihe aufweist, wobei die Magnetreihen jeweils in einer Umfangsrichtung des ersten Lagerrings zueinander beabstandet angeordnete Elektromagnete aufweisen, wobei die Elektromagnete der Magnetreihen derart ausgerichtet sind, dass sie jeweils eine magnetische Kraft auf den zweiten Lagerring bewirken, die quer zu der Drehachse und quer zu einer senkrecht zu der Drehachse angeordneten Radialebene ausgerichtet ist.

Bei dem Verfahren können dieselben Vorteile erreicht werden, die bereits im Zusammenhang mit dem erfindungsgemäßen Magnetlager beschrieben worden sind.

Zudem können die im Zusammenhang mit dem Magnetlager beschriebenen vorteilhaften Merkmale und Ausgestaltungen auch bei dem Verfahren zum Betrieb des Magnetlagers Anwendung finden.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den Erfindungsgedanken nicht einschränken.

### Kurze Beschreibung der Figuren

Die **Figur 1** zeigt ein erstes Ausführungsbeispiel eines erfindungsgemäßen Magnetlagers in einer perspektivischen Darstellung.
Die **Figur 2** zeigt das erste Ausführungsbeispiel des Magnetlagers in einer seitlichen Ansicht.
Die **Figur 3** zeigt das erste Ausführungsbeispiel des Magnetlagers in einer Schnittdarstellung entlang der in Figur 2 gezeigten Schnittebene III-III.
Die **Figur 4** zeigt eine Detaildarstellung des in Figur 3 mit IV bezeichneten Bereichs des Magnetlagers.
Die **Figur 5** zeigt eine Schnittdarstellung des Magnetlagers gemäß dem ersten Ausführungsbeispiel entlang der in Figur 3 gezeigten Schnittebene V-V.
Die **Figur 6** zeigt ein zweites Ausführungsbeispiel eines erfindungsgemäßen Magnetlagers in einer perspektivischen Darstellung.
Die **Figur 7** zeigt das zweite Ausführungsbeispiel des Magnetlagers in einer seitlichen Ansicht.
Die **Figur 8** zeigt das zweite Ausführungsbeispiel des Magnetlagers in einer Schnittdarstellung entlang der in Figur 7 gezeigten Schnittebene VIII-VIII.
Die **Figur 9** zeigt eine Detaildarstellung des in Figur 8 mit IX bezeichneten Bereichs des Magnetlagers.
Die **Figur 10** zeigt eine Schnittdarstellung des Magnetlagers gemäß dem zweiten Ausführungsbeispiel entlang der in Figur 8 gezeigten Schnittebene X-X.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

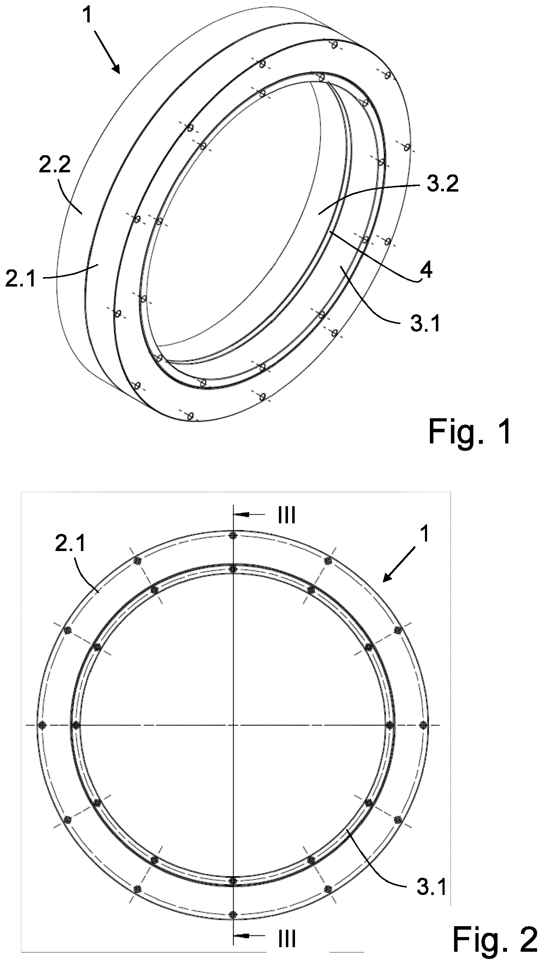

In den **Figuren 1 bis 5** ist ein erstes Ausführungsbeispiel eines Magnetlagers 1 gemäß der Erfindung dargestellt. Das Magnetlager 1 kann in einer Röntgen-Computertomographievorrichtung zur beweglichen Lagerung einer Röntgenquelle und/oder eines Röntgendetektors Verwendung finden. Das Magnetlager 1 weist einen ersten Lagerring 2 auf, der als Außenring ausgestaltet ist. Der erste Lagerring 2 weist zwei Lagerringteile 2.1, 2.2 auf. Die Lagerringteile 2.1, 2.2 sind ringförmig ausgestaltet und können lösbar miteinander verbunden werden, beispielsweise über eine Schraubverbindung. Insofern handelt es sich bei dem ersten Lagerring 2 um einen geteilten Lagerring. Konzentrisch innerhalb des ersten Lagerrings 2 ist ein zweiter Lagerring 3 angeordnet. Der zweite Lagerring 3 weist ebenfalls zwei Lagerringteile 3.1, 3.2 auf. Diese Lagerringteile 3.1, 3.2 sind ringförmig ausgestaltet. Zwischen den Lagerringteilen 3.1, 3.2 des zweiten Lagerrings 3 ist ein Fanglagerteil 4 angeordnet, auf welches noch eingegangen werden wird. Somit handelt es sich bei dem ersten Lagerring 2 um einen Außenring und bei dem zweiten Lagerring 3 um einen Innenring des Magnetlagers 1.

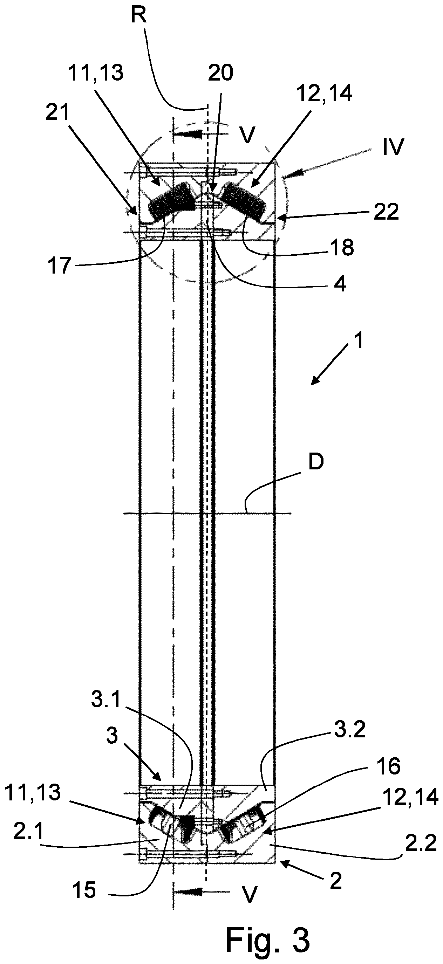

Die beiden Lagerringe 2, 3 sind mittels Elektromagneten 13, 14 um eine Drehachse drehbar zueinander gelagert. Die Elektromagnete 13, 14 dieses Magnetlagers 1 sind in genau zwei Magnetreihen 11, 12 angeordnet. Eine erste Magnetreihe 11 wird durch die in der Figur 3 links gezeigten Elektromagnete 13 gebildet, welche in der Umfangsrichtung des ersten Lagerrings 2 beabstandet zueinander angeordnet sind. Die Elektromagnete 14, die in der Figur 3 rechts gezeigt sind, bilden eine zweite Magnetreihe 12. Auch die Elektromagnete 14 der zweiten Magnetreihe 12 sind in der Umfangsrichtung beabstandet zueinander angeordnet. Die erste und die zweite Magnetreihe 11, 12 können eine identische Anzahl an Elektromagneten 13, 14 aufweisen. Im vorliegenden Fall weisen die Magnetreihen 11, 12 jeweils 16 Elektromagnete 13, 14 auf. Alternativ kann die Anzahl der Elektromagnete 13, 14 pro Magnetreihe 11, 12 beispielsweise vier, acht, 12, 14, 18, 20, 22 oder 24 sein.

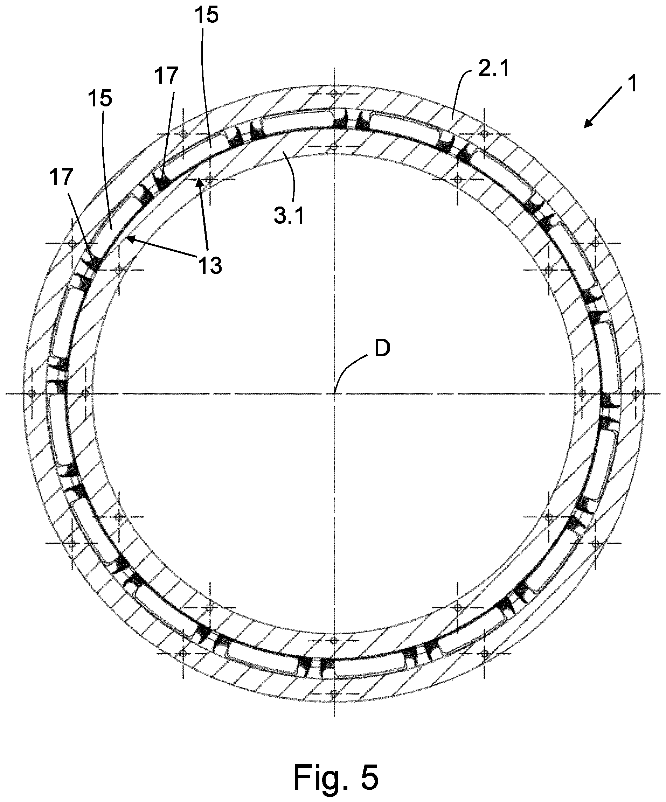

Die zueinander beabstandete Anordnung der Elektromagnete 13 der ersten Magnetreihe 11 innerhalb des ersten Lagerrings 2 kann insbesondere der Darstellung in Fig. 5 entnommen werden. Hierzu sind in dem ersten Lagerringteil 2.1 des ersten Lagerrings 2 eine oder mehrere Aufnahmen für die Spulen 17 der Elektromagnete 13 vorgesehen. Die Spulenkerne 15 der Elektromagnete sind bevorzugt einstückig mit dem ersten Lagerring 2, insbesondere dem ersten Lagerringteil 2.1, ausgebildet.

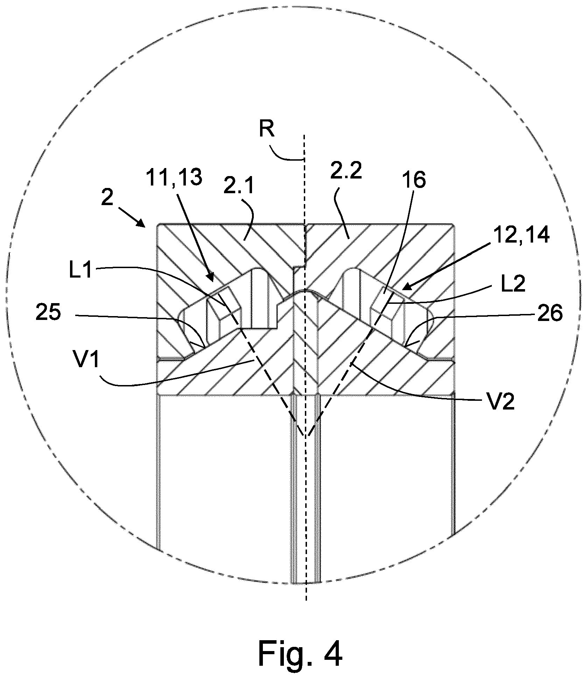

Bei dem Magnetlager 1 sind besondere Vorkehrungen getroffen, um eine möglichst kostengünstige Fertigung zu ermöglichen. Hierzu sind die Elektromagnete 13, 14 der Magnetreihen 11, 12 derart ausgerichtet, dass sie jeweils eine magnetische Kraft auf den zweiten Lagerring 3 bewirken können, die quer zu der Drehachse D und quer zu einer senkrecht zu der Drehachse D angeordneten Radialebene R ausgerichtet ist. Die Elektromagnete 13, 14 der ersten und zweiten Magnetreihe 11, 12 sind also derart angeordnet, dass sie eine magnetische Kraft auf an dem zweiten Lagerring 3 vorgesehene Wirkflächen 25, 26 bewirken können, die Kraftkomponenten sowohl in axialer Richtung als auch in radialer Richtung aufweist, vgl. Figur 4. Diese Wirkflächen 25, 26 sind quer zu der Drehachse D und quer zu der Radialebene R ausgerichtet. Eine dritte Magnetreihe ist bei dem Magnetlager 1 weder erforderlich noch vorgesehen.

Wie die Detaildarstellung in Figur 4 zeigt, umfassen die Elektromagnete 13, 14 der Magnetreihen 11, 12 jeweils eine Spule, die um einen Spulenkern 15, 16 gewickelt ist. Der Spulenkern 15, 16 weist jeweils eine Längsachse L1, L2 auf, die in Richtung eines Luftspalts zwischen dem ersten Lagerring 2 und dem zweiten Lagerring 3 verläuft. Dabei ist die Längsachse L1, L2 des Spulenkerns quer zu der Drehachse D und quer zu der Radialebene R ausgerichtet. Bei dem Magnetlager des ersten Ausführungsbeispiels sind die die Elektromagnete 13, 14 der Magnetreihen 11, 12 derart ausgerichtet, dass eine gedachte Verlängerung V1 der Längsachse L1 des Spulenkerns 15 eines Elektromagneten 13 der ersten Magnetreihe 11 ausgehend von dem ersten Lagerring 2 und in Richtung des zweiten Lagerrings 3 und eine gedachte Verlängerung V2 der Längsachse L2 des Spulenkerns 16 eines Elektromagneten 14 der zweiten Magnetreihe 12 ausgehend von dem ersten Lagerring 2 und in Richtung des zweiten Lagerrings 3 eine zwischen der ersten und zweiten Magnetreihe angeordnete Radialebene R schneiden. Insofern laufen die beiden Verlängerungen V1 und V2 der Längsachsen L1, L2 in Richtung des zweiten Lagerrings 3 aufeinander zu. Es ergibt sich ein im Wesentlichen dreieckiger Querschnitt des zweiten Lagerrings 3 entlang der axialen Schnittebene, vgl. Figur 3 und 4.

Die Elektromagnete 13 der ersten Magnetreihe 11 sind bezüglich der Radialebene R symmetrisch zu den Elektromagneten 14 der zweiten Magnetreihe 12 angeordnet. Die Längsachsen L1, L2 der Elektromagneten 13, 14 können beispielsweise eine Neigung gegenüber der Radialebene R aufweisen, die im Bereich von 5° bis 85°, bevorzugt von 15° bis 75°, besonders bevorzugt von 30° bis 60°, beispielsweise von 45° beträgt.

Der zweite Lagerring 3 des Magnetlagers 1 weist ferner eine Flusstrennung 4 aus einem nicht-magnetischen Material auf, welche ringförmig ausgebildet ist. Die Flusstrennung 4 kann beispielsweise aus Aluminium, Austenit-Stahl, Bronze oder einer Keramik ausgebildet sein. Die Flusstrennung 4 ist bezogen auf das Magnetlager 1 zwischen der ersten Magnetreihe 11 und der zweiten Magnetreihe 12 angeordnet und kann daher die Magnetkreise dieser beiden Magnetreihen 11, 12 wirksam entkoppeln. Die Flusstrennung 4 erfüllt zudem die Funktion eines Fanglagers 20. Hierzu ist die dem Luftspalt zugewandte Oberfläche der Flusstrennung 4 als Fanglagerteil ausgestaltet. Durch die Flusstrennung 4 und den der Flusstrennung 4 gegenüberliegenden Bereich des ersten Lagerrings 2 wird ein als Gleitlager ausgebildetes Fanglager 20 gebildet. Dieses Fanglager 20 befindet sich zwischen der ersten Magnetreihe 11 und der zweiten Magnetreihe 12.

Zusätzlich zu dem ersten Fanglager 20 ist bei dem Magnetlager 1 gemäß dem ersten Ausführungsbeispiel ein zweites Fanglager 21 und ein drittes Fanglager 22 vorgesehen. Diese Fanglager 21, 22 sind ebenfalls als Gleitlager, insbesondere mit abgerundeten Oberflächen, ausgebildet. Das zweite Fanglager 21 und das dritte Fanglager 22 sind derart angeordnet, dass die erste und die zweite Magnetreihe 11, 12 in Richtung der Drehachse D gesehen zwischen dem zweiten Fanglager 21 und einem dritten Fanglager 22 angeordnet sind.

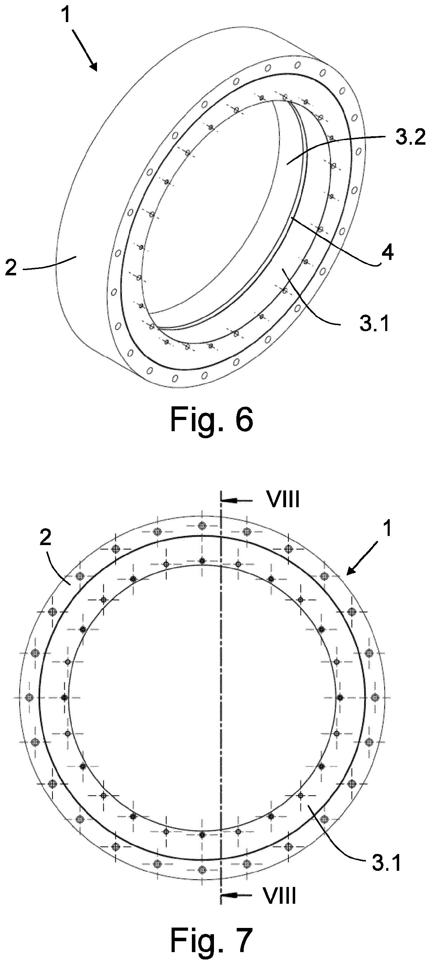

In den **Figuren 6 bis 10** ist ein zweites Ausführungsbeispiel eines Magnetlagers 1 gemäß der Erfindung dargestellt, welches gleichfalls zum Einsatz in einer Röntgen-Computertomographievorrichtung geeignet ist. Dieses Magnetlager 1 weist einen ersten Lagerring 2 auf, der als Außenring ausgestaltet ist. Der erste Lagerring 2 ist einstückig ausgebildet. Alternativ kann der erste Lagerring als geteilter Lagerring ausgebildet sein. Konzentrisch innerhalb des ersten Lagerrings 2 ist ein zweiter Lagerring 3 angeordnet. Der zweite Lagerring 3 weist zwei Lagerringteile 3.1, 3.2 auf. Diese Lagerringteile 3.1, 3.2 sind ringförmig ausgestaltet. Zwischen den Lagerringteilen 3.1, 3.2 des zweiten Lagerrings ist ein Fanglagerteil 4 angeordnet. Insofern handelt es sich bei dem zweiten Lagerring 3 um einen geteilten Innenring.

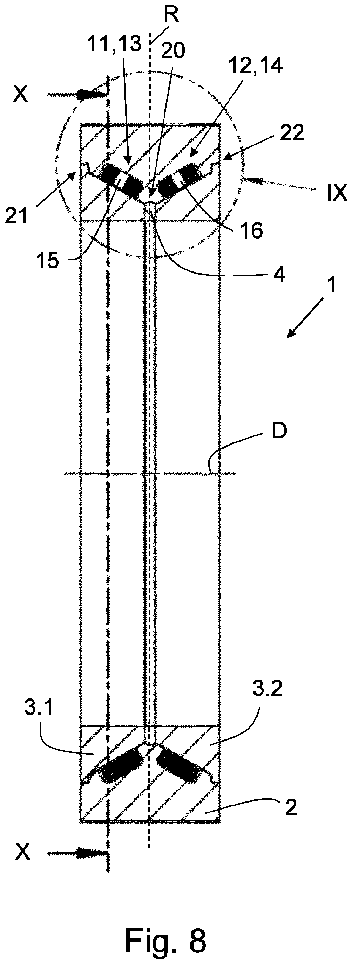

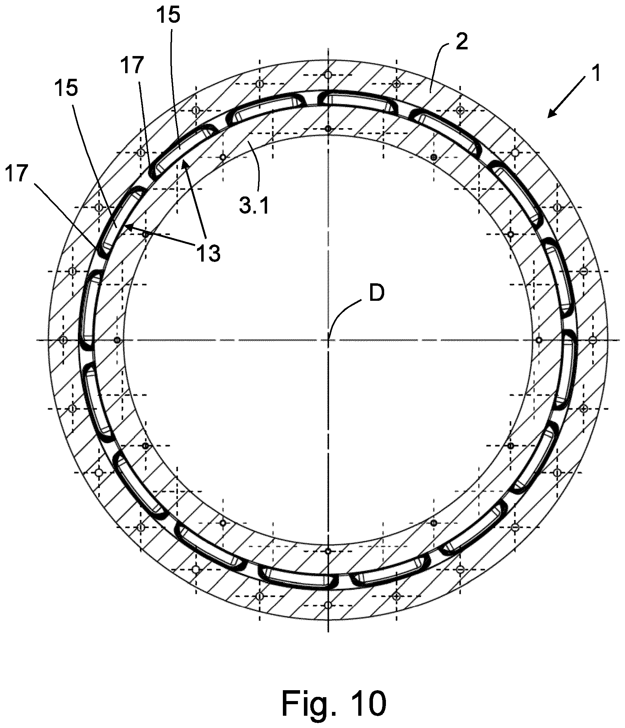

Die beiden Lagerringe 2, 3 sind mittels Elektromagneten 13, 14 um eine Drehachse drehbar zueinander gelagert. Die Elektromagnete 13, 14 dieses Magnetlagers 1 sind in genau zwei Magnetreihen 11, 12 angeordnet. Eine erste Magnetreihe 11 wird durch die in der Figur 8 links gezeigten Elektromagneten 13 gebildet, welche in der Umfangsrichtung des ersten Lagerrings 2 beabstandet zueinander angeordnet sind, wie dies beispielsweise in Figur 10 erkennbar ist. Die Elektromagnete 14, die in der Figur 8 rechts gezeigt sind, bilden eine zweite Magnetreihe 12. Auch die Elektromagnete 14 der zweiten Magnetreihe 12 sind in der Umfangsrichtung beabstandet zueinander angeordnet. Die erste und die zweite Magnetreihe 11, 12 können eine identische Anzahl an Elektromagneten 13, 14 aufweisen, beispielsweise 16 Elektromagnete. Alternativ kann die Anzahl der Elektromagnete 13, 14 pro Magnetreihe 11, 12 vier, acht, 12, 14, 18, 20, 22, 24 oder ein anderer Wert sein.

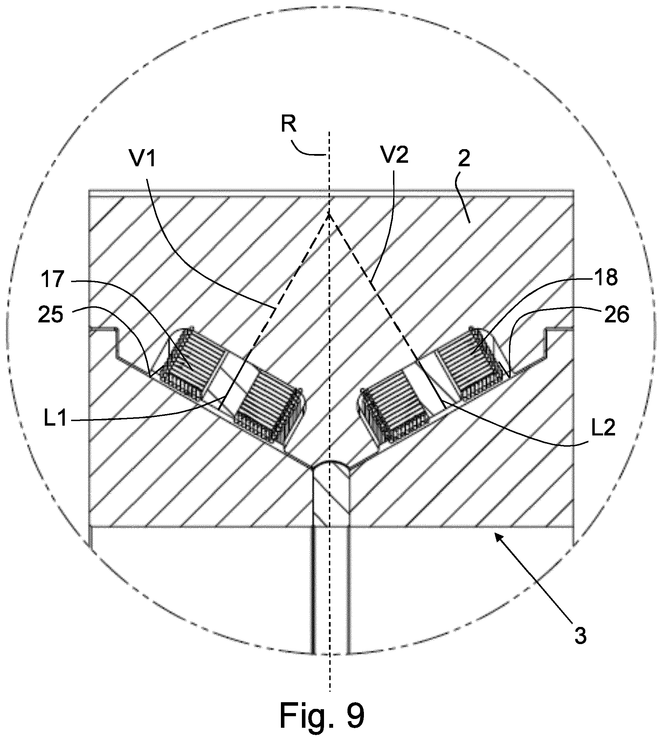

Die Elektromagnete 13, 14 der Magnetreihen 11, 12 weisen jeweils eine Spule 17, 18 und einen Spulenkern 15, 16 auf. Die Elektromagnete 13, 14 sind derart ausgerichtet, dass sie jeweils eine magnetische Kraft auf den zweiten Lagerring 3 bewirken können, die quer zu der Drehachse D und quer zu einer senkrecht zu der Drehachse D angeordneten Radialebene R ausgerichtet ist. Die Elektromagnete 13, 14 der ersten und zweiten Magnetreihe 11, 12 sind also derart angeordnet, dass sie eine magnetische Kraft auf an dem zweiten Lagerring 3 vorgesehene Wirkflächen 25, 26 bewirken können, die Kraftkomponenten sowohl in axialer Richtung als auch in radialer Richtung aufweist, vgl. Figur 9. Diese Wirkflächen 25, 26 sind quer zu der Drehachse D und quer zu der Radialebene R ausgerichtet. Eine dritte Magnetreihe ist bei dem Magnetlager 1 weder erforderlich noch vorgesehen. Der Spulenkern 15, 16 weist jeweils eine Längsachse L1, L2 auf, die in Richtung eines Luftspalts zwischen dem ersten Lagerring 2 und dem zweiten Lagerring 3 verläuft. Dabei ist die Längsachse L1, L2 des Spulenkerns quer zu der Drehachse D und quer zu der Radialebene R ausgerichtet.

Im Unterschied zu dem ersten Ausführungsbeispiel sind bei dem Magnetlager 1 gemäß dem zweiten Ausführungsbeispiel die Elektromagnete 13, 14 der Magnetreihen 11, 12 derart ausgerichtet, dass eine gedachte Verlängerung V1 der Längsachse L1 des Spulenkerns 15 eines Elektromagneten 13 der ersten Magnetreihe 11 ausgehend von dem ersten Lagerring 2 und von dem zweiten Lagerring 3 weg weisend und eine gedachte Verlängerung V2 der Längsachse L2 des Spulenkerns 16 eines Elektromagneten 14 der zweiten Magnetreihe 12 ausgehend von dem ersten Lagerring 2 und von dem zweiten Lagerring 3 weg weisend eine zwischen der ersten und zweiten Magnetreihe angeordnete Radialebene R schneiden. Insofern laufen die beiden von der Drehachse D weg gerichteten Verlängerungen V1 und V2 der Längsachsen L1, L2 in Richtung aufeinander zu. Die Wirkflächen 25, 26 sind an dem zweiten Lagerring 3 derart angeordnet, dass dieser einen im Wesentlichen V-förmigen Querschnitt entlang einer axialen Schnittebene aufweist.

Ähnlich wie bei dem ersten Ausführungsbeispiel sind die Elektromagnete 13 der ersten Magnetreihe 11 bezüglich der Radialebene R symmetrisch zu den Elektromagneten 14 der zweiten Magnetreihe 12 angeordnet. Die Längsachsen L1, L2 der Elektromagneten 13, 14 können beispielsweise eine Neigung gegenüber der Radialebene R aufweisen, die im Bereich von 5° bis 85°, bevorzugt von 15° bis 75°, besonders bevorzugt von 30° bis 60°, beispielsweise von 45° beträgt.

Der zweite Lagerring 3 des Magnetlagers 1 weist ferner eine Flusstrennung 4 aus einem nicht-magnetischen Material auf, welche ringförmig ausgebildet ist. Die Flusstrennung 4 kann beispielsweise aus Aluminium, Austenit-Stahl, Bronze oder einer Keramik ausgebildet sein. Die Flusstrennung 4 ist bezogen auf das Magnetlager 1 zwischen der ersten Magnetreihe 11 und der zweiten Magnetreihe 12 angeordnet und kann daher die Magnetkreise dieser beiden Magnetreihen 11, 12 wirksam entkoppeln. Die Flusstrennung 4 erfüllt zudem die Funktion eines Fanglagers 20. Hierzu ist die dem Luftspalt zugewandte Oberfläche der Flusstrennung 4 als Fanglagerteil ausgestaltet. Durch die Flusstrennung 4 und den der Flusstrennung 4 gegenüberliegenden Bereich des ersten Lagerrings 2 wird ein als Gleitlager ausgebildetes Fanglager 20 gebildet. Dieses Fanglager 20 befindet sich zwischen der ersten Magnetreihe 11 und der zweiten Magnetreihe 12.

Zusätzlich zu dem ersten Fanglager 20 ist bei dem Magnetlager 1 gemäß dem zweiten Ausführungsbeispiel ein zweites Fanglager 21 und ein drittes Fanglager 22 vorgesehen. Diese Fanglager 21, 22 sind ebenfalls als Gleitlager ausgebildet. Das zweite Fanglager 21 und das dritte Fanglager 22 sind derart angeordnet, dass die erste und die zweite Magnetreihe 11, 12 in Richtung der Drehachse D gesehen zwischen dem zweiten Fanglager 21 und einem dritten Fanglager 22 angeordnet sind.

Erfindungsgemäß sind die Elektromagnete 13 der ersten Magnetreihe 11 und die Elektromagnete 12 der zweiten Magnetreihe 14 unterschiedlich ausgelegt.

Die vorstehend gezeigten Magnetlager 1 weisen jeweils einen ersten Lagerring 2 und einen konzentrisch zu dem ersten Lagerring 2 angeordneten zweiten Lagerring 3 auf, wobei der erste Lagerring 2 und der zweite Lagerring 3 mittels Elektromagneten 13, 14 um eine Drehachse D drehbar zueinander gelagert sind, wobei der erste Lagerring 2 eine erste Magnetreihe 11 und eine zweite Magnetreihe 12 aufweist, wobei die Magnetreihen 11, 12 jeweils in einer Umfangsrichtung des ersten Lagerrings 2 zueinander beabstandet angeordnete Elektromagnete 13, 14 aufweisen. Die Elektromagnete 13, 14 der Magnetreihen 11, 12 sind zudem derart ausgerichtet, dass sie jeweils eine magnetische Kraft auf den zweiten Lagerring 3 bewirken können, die quer zu der Drehachse D und quer zu einer senkrecht zu der Drehachse D angeordneten Radialebene R ausgerichtet ist.

### Bezugszeichenliste

- 1: Magnetlager
- 2: Lagerring
- 2.1: Lagerringteil
- 2.2: Lagerringteil
- 3: Lagerring
- 3.1: Lagerringteil
- 3.2: Lagerringteil
- 4: Fanglagerteil

- 11: Magnetreihe
- 12: Magnetreihe
- 13: Elektromagnet
- 14: Elektromagnet
- 15: Spulenkern
- 16: Spulenkern
- 17: Spule
- 18: Spule

- 20: Fanglager
- 21: Fanglager
- 22: Fanglager

- 25: Wirkfläche
- 26: Wirkfläche

- D: Drehachse
- L1: Längsachse
- L2: Längsachse
- R: Radialebene
- V1: Verlängerung
- V2: Verlängerung

## Patentansprüche

1. Röntgen-Computertomographievorrichtung, bei der eine Röntgenquelle und/oder ein Röntgendetektor gegenüber einem zu untersuchenden Objekt über ein Magnetlager mit einem ersten Lagerring (2) und einem konzentrisch zu dem ersten Lagerring (2) angeordneten zweiten Lagerring (3) bewegbar gelagert ist, wobei der erste Lagerring (2) und der zweite Lagerring (3) mittels Elektromagneten (13, 14) um eine Drehachse (D) drehbar zueinander gelagert sind, wobei der erste Lagerring (2) eine erste Magnetreihe (11) und eine zweite Magnetreihe (12) aufweist, wobei die Magnetreihen (11, 12) jeweils in einer Umfangsrichtung des ersten Lagerrings (2) zueinander beabstandet angeordnete Elektromagnete (13, 14) aufweisen, wobei die Elektromagnete (13, 14) der Magnetreihen (11, 12) derart ausgerichtet sind, dass sie jeweils eine magnetische Kraft auf den zweiten Lagerring (3) bewirken können, die quer zu der Drehachse (D) und quer zu einer senkrecht zu der Drehachse (D) angeordneten Radialebene (R) ausgerichtet ist,
**dadurch gekennzeichnet, dass** die Elektromagnete (13) der ersten Magnetreihe (11) bezüglich einer Radialebene (R) asymmetrisch zu den Elektromagneten (14) der zweiten Magnetreihe (12) angeordnet sind und/oder die Elektromagnete (13) der ersten Magnetreihe (11) und die Elektromagnete (14) der zweiten Magnetreihe (12) unterschiedlich ausgelegt sind, wobei die Elektromagnete (13, 14) der ersten und zweiten Magnetreihe (11, 12) unterschiedliche Wicklungszahlen, oder eine unterschiedliche Größe der Spule und/oder des Spulenkerns, oder eine unterschiedliche Breite und/ oder Höhe und/oder Länge, oder unterschiedliche Dimensionierung ihrer Polschuhe aufweisen.

2. Röntgen-Computertomographievorrichtung nach Anspruch 1, wobei das Magnetlager außer den Elektromagneten (13,14) der ersten Magnetreihe (11) und der zweiten Magnetreihe (12) keine weiteren Elektromagneten aufweist.

3. Röntgen-Computertomographievorrichtung nach einem der vorhergehenden Ansprüche, wobei die Elektromagnete (13, 14) der Magnetreihen (11, 12) jeweils eine Spule umfassen, die um einen Spulenkern (15, 16) gewickelt ist, welcher eine Längsachse (L1, L2) aufweist, die in Richtung eines Luftspalts zwischen dem ersten und dem zweiten Lagerring (2, 3) verläuft, wobei die Längsachse (L1, L2) des Spulenkerns (15, 16) quer zu der Drehachse (D) und quer zu der Radialebene (R) ausgerichtet ist.

4. Röntgen-Computertomographievorrichtung nach Anspruch 3, wobei die Elektromagnete (13, 14) der Magnetreihen (11, 12) derart ausgerichtet sind, dass eine gedachte Verlängerung (V1) der Längsachse (L1) des Spulenkerns (15) eines Elektromagneten (13) der ersten Magnetreihe (11) ausgehend von dem ersten Lagerring (2) und in Richtung des zweiten Lagerrings (3) und eine gedachte Verlängerung (V2) der Längsachse (L2) des Spulenkerns (16) eines Elektromagneten (14) der zweiten Magnetreihe (12) ausgehend von dem ersten Lagerring (2) und in Richtung des zweiten Lagerrings (3) eine zwischen der ersten und zweiten Magnetreihe (11, 12) angeordnete Radialebene (R) schneiden.

5. Röntgen-Computertomographievorrichtung nach Anspruch 3, wobei die Elektromagnete (13, 14) der Magnetreihen (11, 12) derart ausgerichtet sind, dass eine gedachte Verlängerung (V1) der Längsachse (L1) des Spulenkerns (15) eines Elektromagneten (13) der ersten Magnetreihe (11) ausgehend von dem ersten Lagerring (2) und von dem zweiten Lagerring (3) weg weisend und eine gedachte Verlängerung der Längsachse (L2) des Spulenkerns (16) eines Elektromagneten (14) der zweiten Magnetreihe (12) ausgehend von dem ersten Lagerring (2) und von dem zweiten Lagerring (3) weg weisend eine zwischen der ersten und zweiten Magnetreihe (11, 12) angeordnete Radialebene (R) schneiden.

6. Röntgen-Computertomographievorrichtung nach einem der vorhergehenden Ansprüche, wobei der zweite Lagerring (3) eine, insbesondere ringförmige, Flusstrennung (4) aus einem nicht-magnetischen Material aufweist.

7. Röntgen-Computertomographievorrichtung nach einem der vorhergehenden Ansprüche, wobei das Magnetlager (1) mindestens ein als Gleitlager ausgebildetes Fanglager (20, 21, 22) aufweist.

8. Röntgen-Computertomographievorrichtung nach Anspruch 7, wobei das Fanglager (20) zwischen der ersten und der zweiten Magnetreihe (11, 12) angeordnet ist.

9. Röntgen-Computertomographievorrichtung nach einem der Ansprüche 7 oder 8, wobei die erste und die zweite Magnetreihe (11, 12) in Richtung der Drehachse (D) gesehen zwischen einem zweiten Fanglager (21) und einem dritten Fanglager (22) angeordnet sind.

10. Röntgen-Computertomographievorrichtung nach einem der vorhergehenden Ansprüche, wobei der erste Lagerring (2) ein Außenring ist und der zweite Lagerring (3) ein Innenring ist.

11. Röntgen-Computertomographievorrichtung nach einem der Ansprüche 1 bis 9, wobei der erste Lagerring (2) ein Innenring und der zweite Lagerring (3) ein Außenring ist.

12. Röntgen-Computertomographievorrichtung nach einem der vorhergehenden Ansprüche, wobei der erste Lagerring (2) als geteilter Lagerring mit einem ersten ringförmigen Lagerringteil (2.1) und einem zweiten ringförmigen Lagerringteil (2.2) ausgebildet ist.

13. Röntgen-Computertomographievorrichtung nach einem der vorhergehenden Ansprüche, wobei der zweite Lagerring (3) als geteilter Lagerring mit einem dritten ringförmigen Lagerringteil (3.1) und einem vierten ringförmigen Lagerringteil (3.2) ausgebildet ist.

14. Röntgen-Computertomographievorrichtung nach einem der vorhergehenden Ansprüche, wobei der erste Lagerring und/oder der zweite Lagerring mehrere Bleche aufweist, die gegeneinander elektrisch isoliert sind.

15. Verfahren zum Betrieb einer Röntgen-Computertomographievorrichtung, bei der eine Röntgenquelle und/oder ein Röntgendetektor gegenüber einem zu untersuchenden Objekt über ein Magnetlager (1) mit einem ersten Lagerring (2) und einem konzentrisch zu dem ersten Lagerring (2) angeordneten zweiten Lagerring (3) bewegbar gelagert ist, wobei der erste Lagerring (2) und der zweite Lagerring (3) mittels Elektromagneten (13, 14) um eine Drehachse (D) drehbar zueinander gelagert sind, wobei der erste Lagerring (2) eine erste Magnetreihe (11) und eine zweite Magnetreihe (12) aufweist, wobei die Magnetreihen (11, 12) jeweils in einer Umfangsrichtung des ersten Lagerrings (2) zueinander beabstandet angeordnete Elektromagnete (13, 14) aufweisen, wobei die Elektromagnete (13, 14) der Magnetreihen (11, 12) derart ausgerichtet sind, dass sie jeweils eine magnetische Kraft auf den zweiten Lagerring (3) bewirken, die quer zu der Drehachse (D) und quer zu einer senkrecht zu der Drehachse (D) angeordneten Radialebene (R) ausgerichtet ist,
**dadurch gekennzeichnet, dass** die Elektromagnete (13) der ersten Magnetreihe (11) bezüglich einer Radialebene (R) asymmetrisch zu den Elektromagneten (14) der zweiten Magnetreihe (12) angeordnet sind und/oder die Elektromagnete (13) der ersten Magnetreihe (11) und die Elektromagnete (14) der zweiten Magnetreihe (12) unterschiedlich ausgelegt sind, wobei die Elektromagnete (13, 14) der ersten und zweiten Magnetreihe (11, 12) unterschiedliche Wicklungszahlen, oder eine unterschiedliche Größe der Spule und/oder des Spulenkerns, oder eine unterschiedliche Breite und/oder Höhe und/oder Länge, oder unterschiedliche Dimensionierung ihrer Polschuhe aufweisen.

## Claims

1. An X-ray computed tomography device, in which an X-ray source and/or an X-ray detector is moveably mounted with respect to an object to be investigated by means of a magnetic bearing having a first bearing ring (2) and a second bearing ring (3), which is arranged concentrically in relation to the first bearing ring (2), wherein the first bearing ring (2) and the second bearing ring (3) are mounted by means of electromagnets (13, 14) so as to be rotatable with respect to each other about an axis of rotation (D), wherein the first bearing ring (2) comprises a first magnet row (11) and a second magnet row (12), wherein the magnet rows (11, 12) each comprise electromagnets (13, 14) arranged at a distance from one another in a circumferential direction of the first bearing ring (2), wherein the electromagnets (13, 14) of the magnet rows (11, 12) are oriented such that they can each exert a magnetic force on the second bearing ring (3), which magnetic force is oriented transversely to the axis of rotation (D) and transversely to a radial plane (R) which is arranged perpendicularly to the axis of rotation (D), **characterized in that** the electromagnets (13) of the first magnet row (11), with respect to a radial plane (R), are arranged asymmetrically to the electromagnets (14) of the second magnet row (12) and/or the electromagnets (13) of the first magnet row (11) and the electromagnets (14) of the second magnet row (12) are of a differing design, wherein the electromagnets (13, 14) of the first and the second magnet rows (11, 12) have a different number of turns, or a different coil size and/or a different coil core size, or a different width and/or height and/or length, or a different dimensioning of their pole shoes.

2. The X-ray computed tomography device as claimed in claim 1, wherein the magnetic bearing, other than the electromagnets (13, 14) of the first magnet row (11) and the second magnet row (12), comprises no further electromagnets.

3. The X-ray computed tomography device as claimed in one of the preceding claims, wherein the electromagnets (13, 14) of the magnet rows (11, 12) respectively comprise a coil, which is wound about a coil core (15, 16), having a longitudinal axis (L1, L2) which lies in the direction of an air gap between the first and the second bearing ring (2, 3), wherein the longitudinal axis (L1, L2) of the coil core (15, 16) is oriented transversely to the axis of rotation (D) and transversely to the radial plane (R).

4. The X-ray computed tomography device as claimed in claim 3, wherein the electromagnets (13, 14) of the magnet rows (11, 12) are oriented such that a notional extension (V1) of the longitudinal axis (L1) of the coil core (15) of an electromagnet (13) in the first magnet row (11), originating from the first bearing ring (2) in the direction of the second bearing ring (3), and a notional extension (V2) of the longitudinal axis (L2) of the coil core (16) of an electromagnet (14) in the second magnet row (12), originating from the first bearing ring (2) in the direction of the second bearing ring (3), intersect with a radial plane (R) which is arranged between the first and the second magnet row (11, 12).

5. The X-ray computed tomography device as claimed in claim 3, wherein the electromagnets (13, 14) of the magnet rows (11, 12) are oriented such that a notional extension (V1) of the longitudinal axis (L1) of the coil core (15) of an electromagnet (13) in the first magnet row (11), originating from the first bearing ring (2) and extending away from the second bearing ring (3), and a notional extension of the longitudinal axis (L2) of the coil core (16) of an electromagnet (14) in the second magnet row (12), originating from the first bearing ring (2) and extending away from the second bearing ring (3), intersect with a radial plane (R) which is arranged between the first and the second magnet row (11, 12).

6. The X-ray computed tomography device as claimed in one of the preceding claims, wherein the second bearing ring (3) comprises a, particularly annular, flux separator (4) of a non-magnetic material.

7. The X-ray computed tomography device as claimed in one of the preceding claims, wherein the magnetic bearing (1) comprises at least one back-up bearing (20, 21, 22) which is configured as a plain bearing.

8. The X-ray computed tomography device as claimed in claim 7, wherein the back-up bearing (20) is arranged between the first and the second magnet row (11, 12).

9. The X-ray computed tomography device as claimed in one of claims 7 or 8, wherein the first and the second magnet row (11, 12), considered in the direction of the axis of rotation (D), is arranged between a second back-up bearing (21) and a third back-up bearing (22).

10. The X-ray computed tomography device as claimed in one of the preceding claims, wherein the first bearing ring (2) is an outer ring, and the second bearing ring (3) is an inner ring.

11. The X-ray computed tomography device as claimed in one of claims 1 to 9, wherein the first bearing ring (2) is an inner ring, and the second bearing ring (3) is an outer ring.

12. The X-ray computed tomography device as claimed in one of the preceding claims, wherein the first bearing ring (2) is configured as a sectional bearing ring having a first annular bearing ring part (2.1) and a second annular bearing ring part (2.2).

13. The X-ray computed tomography device as claimed in one of the preceding claims, wherein the second bearing ring (3) is configured as a sectional bearing ring having a third annular bearing ring part (3.1) and a fourth annular bearing ring part (3.2).

14. The X-ray computed tomography device as claimed in one of the preceding claims, wherein the first bearing ring and/or the second bearing ring comprises a plurality of sheet metal plates which are mutually electrically insulated.

15. A method for operating an X-ray computed tomography device, in which an X-ray source and/or an X-ray detector is moveably mounted with respect to an object to be investigated by means of a magnetic bearing (1) having a first bearing ring (2) and a second bearing ring (3), which is arranged concentrically in relation to the first bearing ring (2), wherein the first bearing ring (2) and the second bearing ring (3) are mounted by means of electromagnets (13, 14) so as to be rotatable with respect to each other about an axis of rotation (D), wherein the first bearing ring (2) has a first magnet row (11) and a second magnet row (12), wherein the magnet rows (11, 12) each comprise electromagnets (13, 14) arranged at a distance from one another in a circumferential direction of the first bearing ring (2), wherein the electromagnets (13, 14) of the magnet rows (11, 12) are oriented such that they can each exert a magnetic force on the second bearing ring (3), which magnetic force is oriented transversely to the axis of rotation (D) and transversely to a radial plane (R) which is arranged perpendicularly to the axis of rotation (D), **characterized in that** the electromagnets (13) of the first magnet row (11), with respect to a radial plane (R), are arranged asymmetrically to the electromagnets (14) of the second magnet row (12) and/or the electromagnets (13) of the first magnet row (11) and the electromagnets (14) of the second magnet row (12) are of a differing design, wherein the electromagnets (13, 14) of the first and the second magnet rows (11, 12) have a different number of turns, or a different coil size and/or a different coil core size, or a different width and/or height and/or length, or a different dimensioning of their pole shoes..

## Revendications

1. Dispositif de tomodensitométrie à rayons X, avec lequel une source de rayons X et/ou un détecteur de rayons X est, par rapport à un objet à examiner, monté avec mobilité par le biais d'un palier magnétique ayant une première bague de palier (2) et une deuxième bague de palier (3), disposée de manière concentrique par rapport à la première bague de palier (2), la première bague de palier (2) et la deuxième bague de palier (3) étant montées rotatives autour d'un axe de rotation (D) l'une par rapport à l'autre au moyen d'électroaimants (13, 14), la première bague de palier (2) possédant une première rangée d'aimants (11) et une deuxième rangée d'aimants (12), les rangées d'aimants (11, 12) possédant respectivement des électroaimants (13, 14) disposés espacés les uns des autres dans une direction périphérique de la première bague de palier (2), les électroaimants (13, 14) des rangées d'aimants (11, 12) étant orientés de telle sorte qu'ils peuvent respectivement exercer une force magnétique sur la deuxième bague de palier (3), laquelle est orientée transversalement par rapport à l'axe de rotation (D) et transversalement par rapport à un plan radial (R) disposé perpendiculairement à l'axe de rotation (D), **caractérisé en ce que**
les électroaimants (13) de la première rangée d'aimants (11) sont disposés de manière asymétrique par rapport aux électroaimants (14) de la deuxième rangée d'aimants (12) en référence à un plan radial (R) et/ou les électroaimants (13) de la première rangée d'aimants (11) et les électroaimants (14) de la deuxième rangée d'aimants (12) sont de conceptions différentes, les électroaimants (13, 14) des première et deuxième rangées d'aimants (11, 12) possédant des nombres d'enroulements différents ou une taille différente de la bobine et/ou du noyau de bobine, ou une largeur et/ou une hauteur et/ou une longueur différentes, ou un dimensionnement différent de leurs pièces polaires.

2. Dispositif de tomodensitométrie à rayons X selon la revendication 1, le palier magnétique ne possédant aucun électroaimant supplémentaire à part les électroaimants (13, 14) de la première rangée d'aimants (11) et de la deuxième rangée d'aimants (12).

3. Dispositif de tomodensitométrie à rayons X selon l'une des revendications précédentes, les électroaimants (13, 14) des rangées d'aimants (11, 12) comportant respectivement une bobine, qui est enroulée autour d'un noyau de bobine (15, 16), lequel possède un axe longitudinal (L1, L2) qui suit un tracé en direction d'un entrefer entre la première et la deuxième bague de palier (2, 3), l'axe longitudinal (L1, L2) du noyau de bobine (15, 16) étant orienté transversalement par rapport à l'axe de rotation (D) et transversalement par rapport au plan radial (R).

4. Dispositif de tomodensitométrie à rayons X selon la revendication 3, les électroaimants (13, 14) des rangées d'aimants (11, 12) étant orientés de telle sorte qu'une prolongation imaginaire (V1) de l'axe longitudinal (L1) du noyau de bobine (15) d'un électroaimant (13) de la première rangée d'aimants (11), partant de la première bague de palier (2) et en direction de la deuxième bague de palier (3), et une prolongation imaginaire (V2) de l'axe longitudinal (L2) du noyau de bobine (16) d'un électroaimant (14) de la deuxième rangée d'aimants (12), partant de la première bague de palier (2) et en direction de la deuxième bague de palier (3), croisent un plan radial (R) disposé entre la première et la deuxième rangée d'aimants (11, 12).

5. Dispositif de tomodensitométrie à rayons X selon la revendication 3, les électroaimants (13, 14) des rangées d'aimants (11, 12) étant orientés de telle sorte qu'une prolongation imaginaire (V1) de l'axe longitudinal (L1) du noyau de bobine (15) d'un électroaimant (13) de la première rangée d'aimants (11), partant de la première bague de palier (2) et s'éloignant de la deuxième bague de palier (3), et une prolongation imaginaire de l'axe longitudinal (L2) du noyau de bobine (16) d'un électroaimant (14) de la deuxième rangée d'aimants (12), partant de la première bague de palier (2) et s'éloignant de la deuxième bague de palier (3), croisent un plan radial (R) disposé entre la première et la deuxième rangée d'aimants (11, 12).

6. Dispositif de tomodensitométrie à rayons X selon l'une des revendications précédentes, la deuxième bague de palier (3) possédant une séparation de flux (4), notamment de forme annulaire, en un matériau non magnétique.

7. Dispositif de tomodensitométrie à rayons X selon l'une des revendications précédentes, le palier magnétique (1) possédant au moins un palier de secours (20, 21, 22) réalisé sous la forme d'un palier de glissement.

8. Dispositif de tomodensitométrie à rayons X selon la revendication 7, le palier de secours (20) étant disposé entre la première et la deuxième rangée d'aimants (11, 12) .

9. Dispositif de tomodensitométrie à rayons X selon l'une des revendications 7 et 8, la première et la deuxième rangée d'aimants (11, 12), vues dans la direction de l'axe de rotation (D), étant disposées entre un deuxième palier de secours (21) et un troisième palier de secours (22).

10. Dispositif de tomodensitométrie à rayons X selon l'une des revendications précédentes, la première bague de palier (2) étant une bague extérieure et la deuxième bague de palier (3) étant une bague intérieure.

11. Dispositif de tomodensitométrie à rayons X selon l'une des revendications 1 à 9, la première bague de palier (2) étant une bague intérieure et la deuxième bague de palier (3) étant une bague extérieure.

12. Dispositif de tomodensitométrie à rayons X selon l'une des revendications précédentes, la première bague de palier (2) étant réalisée sous la forme d'une bague de palier divisée avec une première partie de bague de palier (2.1) de forme annulaire et une deuxième partie de bague de palier (2.2) de forme annulaire.

13. Dispositif de tomodensitométrie à rayons X selon l'une des revendications précédentes, la deuxième bague de palier (3) étant réalisée sous la forme d'une bague de palier divisée avec une troisième partie de bague de palier (3.1) de forme annulaire et une quatrième partie de bague de palier (3.2) de forme annulaire.

14. Dispositif de tomodensitométrie à rayons X selon l'une des revendications précédentes, la première bague de palier et/ou la deuxième bague de palier possédant plusieurs tôles qui sont isolées électriquement les unes des autres.

15. Procédé pour faire fonctionner un dispositif de tomodensitométrie à rayons X, avec lequel une source de rayons X et/ou un détecteur de rayons X est, par rapport à un objet à examiner, monté avec mobilité par le biais d'un palier magnétique (1) ayant une première bague de palier (2) et une deuxième bague de palier (3), disposée de manière concentrique par rapport à la première bague de palier (2), la première bague de palier (2) et la deuxième bague de palier (3) étant montées rotatives autour d'un axe de rotation (D) l'une par rapport à l'autre au moyen d'électroaimants (13, 14), la première bague de palier (2) possédant une première rangée d'aimants (11) et une deuxième rangée d'aimants (12), les rangées d'aimants (11, 12) possédant respectivement des électroaimants (13, 14) disposés espacés les uns des autres dans une direction périphérique de la première bague de palier (2), les électroaimants (13, 14) des rangées d'aimants (11, 12) étant orientés de telle sorte qu'ils exercent respectivement une force magnétique sur la deuxième bague de palier (3), laquelle est orientée transversalement par rapport à l'axe de rotation (D) et transversalement par rapport à un plan radial (R) disposé perpendiculairement à l'axe de rotation (D), **caractérisé en ce que**
les électroaimants (13) de la première rangée d'aimants (11) sont disposés de manière asymétrique par rapport aux électroaimants (14) de la deuxième rangée d'aimants (12) en référence à un plan radial (R) et/ou les électroaimants (13) de la première rangée d'aimants (11) et les électroaimants (14) de la deuxième rangée d'aimants (12) sont de conceptions différentes, les électroaimants (13, 14) des première et deuxième rangées d'aimants (11, 12) possédant des nombres d'enroulements différents ou une taille différente de la bobine et/ou du noyau de bobine, ou une largeur et/ou une hauteur et/ou une longueur différentes, ou un dimensionnement différent de leurs pièces polaires.
